# EUROPÄISCHE PATENTSCHRIFT

(11) **EP 1 965 833 B1**
(45) Veröffentlichungstag und Bekanntmachung des Hinweises auf die Patenterteilung: **28.08.2013**
(21) Anmeldenummer: 06841499.4
(22) Anmeldetag: 20.12.2006
(51) Int. Cl.: A61L 2/14, H05H 1/24, H05H 1/48

(54) **VERFAHREN ZUR DESINFEKTION VON GEGENSTÄNDEN**
METHOD FOR THE DISINFECTION OF OBJECTS
PROCEDE POUR DESINFECTER DES OBJETS

(30) Priorität: 20.12.2005 DE 102005061236
(43) Veröffentlichungstag der Anmeldung: 10.09.2008
(73) Patentinhaber: PlasmaTreat GmbH, 33803 Steinhagen (DE); Fraunhofer-Gesellschaft zur Förderung der angewandten Forschung e.V., 80686 München (DE)
(72) Erfinder: BUSKE, Christian, 33803 Steinhagen (DE); FÖRNSEL, Peter, 32139 Spenge (DE); WUNDERLICH, Joachim, 84172 Buch Am Erlbach (DE)
(74) Vertreter: Cohausz & Florack
(86) Internationale Anmeldenummer: PCT/EP2006/069997
(87) Internationale Veröffentlichungsnummer: WO 2007/071720

(56) Entgegenhaltungen:
- EP-A- 1 132 195
- WO-A-01/43512
- WO-A-03/056602
- JP-A- 2004 173 704
- US-A1- 2002 187 066

## Beschreibung

Die Erfindung betrifft ein Verfahren zur Desinfektion von Gegenständen.

Bei vielfältigen industriellen und labortechnischen Verfahren kommt es auf eine gute Desinfektion der benutzten Gegenstände an.

Unter Desinfektion wird die Reduktion der Anzahl lebensfähiger Mikroorganismen mit Hilfe physikalischer und chemischer Methoden verstanden. Ziel der Desinfektion ist die Unschädlichmachung bestimmter pathogener Mikroorganismen durch Eingriffe in deren Struktur oder Stoffwechsel. Unter einer Desinfektion im Rahmen dieser Erfindung wird auch jede Form der Sterilisation und jede Form der Entkeimung verstanden.

Die Anwendungen der Desinfektion sind hauptsächlich Flächen- und Raumdesinfektion, Geräte- und Instrumentendesinfektion und Wäschedesinfektion. Diese Anwendungen liegen vor allem im medizinischen Bereich, jedoch können auch andere nicht-medizinische Anwendungen eine Desinfektion benötigen. Als Beispiel sei die Lebensmittelindustrie genannt, bei der es bei der Entkeimung von Lebensmitteln oder von Aufbewahrungsmitteln für Lebensmittel, bspw. Teller oder Töpfe, auf eine sorgfältige Säuberung und Desinfektion bzw. Entkeimung ankommt.

Die aus dem Stand der Technik bekannten Desinfektionsmittel für Oberflächen sind häufig sehr aggressiv, dass man sie nur unter besonderen Vorsichtsmaßnahmen, beispielsweise mit Schutzhandschuhen verarbeiten darf. Das Einatmen der aus den verwendeten Flüssigkeiten konzentriert entstehenden Dämpfe kann zudem gesundheitliche Schäden verursachen.

Die zuvor genannten desinfizierenden Stoffe werden oft in einer großen Konzentration auf die Oberfläche aufgebracht. Meist obliegt es dem Benutzer, wie viel des desinfizierenden Stoffes er aufbringt. Dabei ist oft eine hohe Konzentration notwendig, weil der desinfizierende Stoff nicht ausreichend wirksam werden kann.

Weitere Desinfektionsmethoden basieren auf der Anwendung von Strahlung wie UV-Strahlung oder sogar radioaktiver Strahlung. Diese Methoden haben aber entweder den Nachteil einer geringen Effektivität oder bedeuten einen hohen technischen Aufwand.

Aus der WO 03/056602 A1 sind eine Vorrichtung und ein Verfahren zur Behandlung einer Werkstückoberfläche mit einem Niedertemperatur-Plasma bekannt, bei denen das Plasma mittels dielektrisch behinderter Entladung erzeugt wird.

Der Erfindung liegt das technische Problem zugrunde, ein Verfahren zur Desinfektion anzugeben, mit dem die zuvor aufgezeigten Nachteile behoben werden.

Das zuvor aufgezeigte technische Problem wird erfindungsgemäß zunächst durch ein Verfahren mit den Merkmalen des Anspruches 1 gelöst. Weitere vorteilhafte Ausgestaltungen sind in den abhängigen Unteransprüchen angegeben.

Beim erfindungsgemäßen Verfahren zur Desinfektion von Gegenständen wird ein atmosphärischer Plasmastrahl unter Anwendung einer elektrischen Entladung in einem von einem Arbeitgas durchströmten Volumen erzeugt und die Oberfläche des Gegenstandes wird mit dem Plasmastrahl zumindest teilweise beaufschlagt. Die desinfizierende Wirkung erfolgt dann durch den Energieübertrag vom Plasmastrahl auf die Oberfläche.

Der Erfindung liegt also die Erkenntnis zugrunde, dass durch Anwendung eines atmosphärischen Plasmastrahls ein reaktives Medium mit der Oberfläche des Gegenstandes in Berührung gebracht wird, das einerseits eine hohe Reaktivität aufgrund höher Elektronenanregung und andererseits eine geringe Gastemperatur aufweist. Die hohe Reaktivität wird für die Desinfektion ausgenutzt, indem die auf den Oberflächen vorhandenen Keime aufgrund der Elektronenreaktivität zumindest teilweise, vorzugsweise überwiegend abgetötet werden.

Gleichzeitig tritt aber durch den Plasmastrahl nur eine geringe thermische Belastung des beaufschlagten Gegenstandes auf, so dass dieser selbst nicht angegriffen, insbesondere verändert wird. Somit können auch empfindliche Oberflächen sorgfältig desinfiziert werden, die bisher nicht mit einem der oben erwähnten aggressiven Desinfektionsmitteln in Berührung gebracht werden konnten.

Ein weiterer Vorteil des erfindungsgemäßen Verfahrens besteht darin, dass der Plasmastrahl anders als die bekannten Anwendungen von flüssigen Desinfektionsmitteln nicht nur glatte Oberflächen, sondern auch schwierig zu erreichende dreidimensionale Strukturen desinfizieren kann, ohne dass Rückstände in Kanten und Ecken verbleiben. Denn der Plasmastrahl sorgt durch die Gasströmung selbst dafür, dass keine Reste einer Verunreinigung in diesen Bereichen haften bleiben.

Die zuvor aufgezeigten Verfahren sowie die nachfolgend beschriebenen Vorrichtungen setzen bevorzugt die nachfolgend beschriebenen Plasmaquellen bzw. Plasmadüsen ein, die das zuvor genannte von einem Arbeitsgas durchströmten Volumen darstellen.

Die Energiezufuhr für die Plasma-Desinfektion wird bevorzugt mit einer Plasmaquelle bzw. Plasmadüse erzeugt, bei der mittels einer unter Anlegen einer hochfrequenten Hochspannung in einem Düsenrohr zwischen zwei Elektroden mittels einer nicht-thermischen Entladung aus einem Arbeitsgas ein Plasmastrahl erzeugt wird. Dabei steht das Arbeitsgas vorzugsweise unter einem Druck in der Größenordnung des Atmosphärendruckes, man spricht daher auch von einem atmosphärischen Plasma.

Der Plasmastrahl tritt aus der Düsenöffnung aus, wobei eine der beiden Elektroden im Bereich der Düsenöffnung angeordnet ist. Der nicht-thermische Plasmastrahl weist bevorzugt außerhalb der Plasmadüse bei einer geeignet eingestellten Strömungsrate keine elektrischen Streamer auf, also Entladungskanäle der elektrischen Entladung, so dass nur der energiereiche, aber niedrig temperierte Plasmastrahl auf die Oberfläche gerichtet wird. Ein solcher atmosphärischer Plasmastrahl wird auch als potentialfreier Plasmastrahl bezeichnet. Die Spannungsdifferenz zwischen der Düsenöffnung und dem Werkstück liegt dabei bevorzugt unterhalb von 100 V.

Zur Charakterisierung der Gaseigenschaften des Plasmastrahls wird von einer hohen Elektronentemperatur und einer niedrigen Ionentemperatur gesprochen. Die hohe Elektronentemperatur bewirkt eine hohe Reaktivität des Plasmagases oder Plasmagasgemisches. Die niedrige Ionentemtemperatur dagegen bewirkt eine geringe Wärmeenergie, die beim Auftreffen des Plasmastrahls auf der Oberfläche auf diese übertragen wird.

Aus dem Stand der Technik der EP 0 761 415 A1 und der EP 1 335 641 A1 sind derartige Plasmaquellen an sich bekannt. Für eine großflächigere Anwendung des Plasmastrahls eignen sich die aus der WO 99/52333 und der WO 01/43512 bekannten Rotationsdüsen.

In bevorzugter Weise wird der Plasmastrahl mit Hilfe einer atmosphärischen Entladung in einem Sauerstoff enthaltenden Arbeitsgas erzeugt. Dadurch wird die Reaktivität des Plasmastrahls erhöht. In bevorzugter Weise wird Luft als Arbeitsgas verwendet. Ebenso kann ein Arbeitsgas aus einer Mischung aus Wasserstoff und Stickstoff eingesetzt werden, ein sogenanntes Formiergas. Als Arbeitsgas kommt auch nur Stickstoff in Frage.

Die nicht-thermische Plasmaentladung erfolgt unter Anwendung einer hochfrequenten Hochspannung, wobei eine Folge von Entladungen zwischen zwei Elektroden der Plasmadüse erzeugt wird und das Arbeitsgas zu einem aus der Plasmadüse austretenden Plasma angeregt wird. Gerade die hochfrequente Folge der Entladungen gewährleistet, dass kein thermisches Gleichgewicht im Entladungsraum entsteht. Somit kann auch im Dauerbetrieb das Ungleichgewicht zwischen Elektronentemperatur und Ionentemperatur aufrecht gehalten werden.

Die Effektivität der Plasmabehandlung hängt natürlich von der Wahl des Prozessgases, der Leistung, der Behandlungsdauer und des Anlagenkonzeptes ab und es können je nach Anforderung Anpassungen vorgenommen werden. Insbesondere stellen die Spannungswerte Frequenz und Amplitude geeignete Mittel dar, um die Effektivität der Plasmabehandlung zu beeinflussen.

Beim Stand der Technik der DE 37 33 492 erfolgt das Erzeugen des atmosphärischen Plasmastrahls mittels einer Koronaentladung durch eine Ionisation eines Arbeitsgases, bspw. Luft. Die Vorrichtung besteht aus einem Keramikrohr, das an der äußeren Wandung mit einer äußeren Elektrode umgeben ist. Mit wenigen Millimetern Abstand zur Innenwandung des Keramikrohres ist eine innere Elektrode als Stab angeordnet. Durch den Spalt zwischen der Innenwandung des Keramikrohres und der inneren Elektrode wird ein ionisierbares Gas wie Luft oder Sauerstoff geleitet. An die beiden Elektroden wird ein hochfrequentes Hochspannungsfeld angelegt, wie es bei einer Koronavorbehandlung von Folien eingesetzt wird. Durch das Wechselfeld wird das durchgeführte Gas ionisiert und tritt am Rohrende aus.

Die zuvor erläuterten Verfahren ermöglichen die oben erwähnte erfindungsgemäße Anwendung zur Desinfektion von Gegenständen.

In bevorzugter Weise kann die sterilisierende Wirkung des Plasmastrahls dadurch erhöht werden, dass dem vom Arbeitsgas durchströmten Volumen ein desinfizierender Stoff zugemischt wird. Dieser desinfizierende Stoff wird durch die Entladung und/oder durch den Plasmastrahl angeregt und kann dann beim Auftreffen auf die Oberfläche des Gegenstandes seine so verstärkte desinfizierende Wirkung ausüben.

Unter Anregung des desinfizierenden Stoffs wird dabei verstanden, dass dieser elektronisch angeregt, ionisiert oder dissoziiert wird. Gerade diese angeregten Zustände ermöglichen einen besonders effektiven Energietransfer vom Plasmastrahl auf die Oberfläche des Gegenstandes und erhöhen damit die desinfizierende Wirkung des Plasmastrahls an sich.

In bevorzugter Weise können als desinfizierender Stoff gasförmige Stoffe dem Arbeitgas oder dem Plasmastrahl zugemischt werden. Des Weiteren können als desinfizierende Stoffe flüssige desinfizierende Stoffe zugemischt werden. Im Falle von flüssigen desinfizierenden Stoffen werden diese bevorzugt in das Volumen, das vom Arbeitsgas durchströmt wird, eingedüst, versprüht oder vernebelt, damit eine möglichst gleichmäßige Verteilung der Flüssigkeit im Arbeitsgas oder im Plasmastrahl erreicht wird.

Der desinfizierende Stoff kann ausgewählt werden aus der folgenden Gruppe:
- Aldehyde, wie Formaldehyd, Glutaraldehyd, Glyoxal.
- Alkohole, wie Ethanol, Propanol, Isopropanol.
- Per-Verbindungen, wie Wasserstoffperoxid, PerEssigsäure, Kaliumperoxomono-sulfat.
- Halogene, wie Natriumhypochlorit, Chlordioxid, Natriumchlorit, Chloramin.
- Halogenierte Phenole, wie m-Kresol, p-Chlor-m-Kresol, p-Chlor-m-Xylenol
- Quaternäre Ammoniumverbindungen, wie Benzalkonium.

Als flüssiger desinfizierender Stoff kann auch Wasser zugemischt werden. Denn durch die Anregung im und/oder durch das Plasma entsteht ein Arbeitsgas/Wasserdampfgemisch, das ebenfalls eine desinfizierende Wirkung aufweist, auch wenn Wasser an sich selbst bei normalen Temperaturen keine desinfizierende Wirkung hat.

Der Einlass des desinfizierenden Stoffes kann grundsätzlich an drei verschiedenen Stellen dem Volumen zugeführt werden.

Zunächst kann der desinfizierende Stoff strömungsaufwärts der Entladungszone dem Arbeitsgas zugemischt werden. Dafür stehen beispielsweise zwei Möglichkeiten zur Verfügung. Zum einen kann der desinfizierende Stoff bereits dem Arbeitsgas zugemischt worden sein, bevor das Arbeitsgas in das Volumen eingelassen wird. Zum anderen kann der desinfizierende Stoff in der Nähe des Einlasses des Arbeitsgases mit einem separaten Einlass eingeführt werden.

Bei einer weiteren Ausgestaltung der Erfindung wird der desinfizierende Stoff im Bereich der Entladungszone dem Arbeitsgas zugemischt wird. Auch hier gibt es zwei Möglichkeiten. Zum einen kann der desinfizierende Stoff seitlich der Entladungszone dem Volumen durch einen separaten Eingang zugeführt. Zum anderen besteht die Möglichkeit, den desinfizierenden Stoff durch eine Bohrung einzulassen, die in einer der für die elektrische Entladung vorgesehenen Elektroden angeordnet ist. Dadurch wird der desinfizierende Stoff sehr genau dem Entladungsbereich zugeführt.

Schließlich kann der desinfizierende Stoff strömungsabwärts der Entladungszone dem Arbeitsgas zugemischt werden. Diese Vorgehensweise ist dann zu bevorzugen, wenn der desinfizierende Stoff nicht der Entladung selber, sondern nur dem weitaus weniger stark einwirkenden Plasmastrahl ausgesetzt werden soll. Dazu kann mittels einer im Plasmastrahl angeordneten Zuleitung oder auch Lanze der desinfizierende Stoff zugemischt werden.

Für die zuvor erläuterten Varianten des Verfahrens können Plasmadüsen eingesetzt werden, wie sie aus dem Stand der Technik bereits für die Plasma-Oberflächenbeschichtung bzw. Plasmapolymerisation bekannt sind. Diesen Düsenformen ist gemeinsam, dass ein für die Plasmapolymerisation notwendiger Precursor dem Volumen der elektrischen Entladung an unterschiedlichen Stellen zugeführt wird. Dazu wird beispielhaft auf die EP 1 230 414 und die DE 100 61 828 hingewiesen.

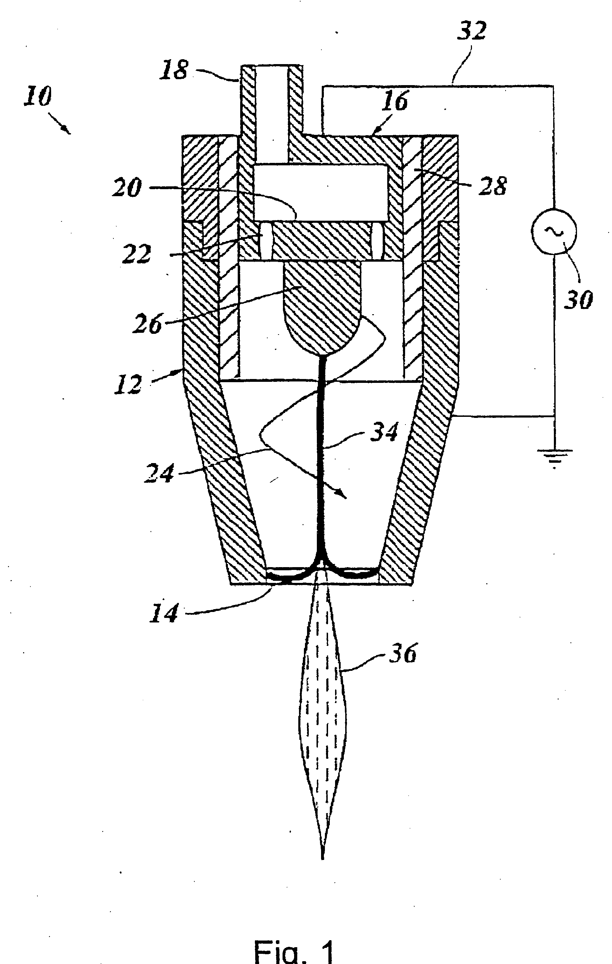

Im Folgenden wird ein Ausführungsbeispiel einer für die Durchführung des erfindungsgemäßen Verfahrens einsetzbare Plasmadüse erläutert, wozu auf die einzige Fig. 1 der Zeichnung verwiesen wird. In dieser Zeichnung zeigt also
- Fig. 1: ein Ausführungsbeispiel einer Plasmadüse, die bei der erfindungsgemäßen Verfahren eingesetzt werden kann.

Eine in Fig. 1 gezeigte Plasmaquelle bzw. Plasmadüse 10 weist ein Düsenrohr 12 aus Metall auf, das sich konisch zu einer Auslassöffnung 14 verjüngt. Am der Auslassöffnung 14 entgegengesetzten Ende weist das Düsenrohr 12 eine Dralleinrichtung 16 mit einen Einlass 18 für ein Arbeitsgas auf, beispielsweise für Druckluft oder Stickstoffgas.

In der Fig. 1 ist die Düse mit einer zentrierten Auslassöffnung dargestellt. Darüber hinaus ist es auch möglich, den Düsenauslass schräg zu stellen und rotierbar gegenüber dem Gehäuse 12 anzuordnen. Dadurch wird eine Rotationsbewegung des Auslasses 14 erreicht, wodurch das Plasma weiter verwirbelt wird. Eine solche Rotationsdüse ist aus der EP 1 067 829 A2 bekannt.

Eine Zwischenwand 20 der Dralleinrichtung 16 weist einen Kranz von schräg in Umfangsrichtung angestellten Bohrungen 22 auf durch die das Arbeitsgas verdrallt wird. Der stromabwärtige, konisch verjüngte Teil des Düsenrohres wird deshalb von dem Arbeitsgas in der Form eines Wirbels 24 durchströmt, dessen Kern auf der Längsachse des Düsenrohres verläuft.

An der Unterseite der Zwischenwand 20 ist mittig eine Elektrode 26 angeordnet, die koaxial in den verjüngten Abschnitt des Düsenrohres hineinragt. Die Elektrode 26 ist elektrisch mit der Zwischenwand 20 und den übrigen Teilen der Dralleinrichtung 16 verbunden. Die Dralleinrichtung 16 ist durch ein Keramikrohr 28 elektrisch gegen das Düsenrohr 12 isoliert. Über die Dralleinrichtung 16 wird an die Elektrode 26 eine hochfrequente Hochspannung, insbesondere Wechselspannung oder eine hochfrequent gepulste Gleichspannung angelegt, die von einem Hochfrequenztransformator 30 erzeugt wird.

Die Primärspannung ist variabel regelbar und beträgt beispielsweise 300 bis 500 V. Die Sekundärspannung kann 1 bis 5 kV oder mehr betragen, gemessen Peak-to-Peak. Die Frequenz liegt beispielsweise in der Größenordnung von 1 bis 100 kHz und ist vorzugsweise ebenfalls regelbar. Die Frequenz kann auch außerhalb der angegebenen Werte eingestellt werden, solange sich eine Bogenentladung einstellt. Die Dralleinrichtung 16 ist mit dem Hochfrequenzgenerator 30 über ein flexibles Hochspannungskabel 32 verbunden. Der Einlass 18 ist über einen nicht gezeigten Schlauch mit einer unter Druck stehenden Arbeitsgasquelle mit variablem Durchsatz verbunden, die vorzugsweise mit dem Hochfrequenzgenerator 30 zu einer Versorgungseinheit kombiniert ist. Das Düsenrohr 12 ist geerdet.

Durch die angelegte Spannung wird eine Hochfrequenzentladung in der Form eines Lichtbogens 34 zwischen der Elektrode 26 und dem Düsenrohr 12 erzeugt. Der Begriff "Lichtbogen" wird als phänomenologische Beschreibung der Entladung verwendet, da die Entladung in Form eines Lichtbogens auftritt, der Begriff Lichtbogen aber bei Gleichspannungsentladungen mit im Wesentlichen konstanten Spannungswerten verstanden wird. Aufgrund der drallförmigen Strömung des Arbeitsgases wird dieser Lichtbogen jedoch im Wirbelkern auf der Achse des Düsenrohres 12 kanalisiert, so dass er sich erst im Bereich der Auslassöffnung 14 zur Wand des Düsenrohres 12 verzweigt. Das Arbeitsgas, das im Bereich des Wirbelkerns und damit in unmittelbarer Nähe des Lichtbogens 34 mit hoher Strömungsgeschwindigkeit rotiert, kommt mit dem Lichtbogen in innige Berührung und wird dadurch zum Teil in den Plasmazustand überführt, so dass ein Strahl 36 eines atmosphärischen Plasmas, etwa in der Gestalt einer Kerzenflamme, aus der Auslassöffnung 14 der Plasmadüse 10 austritt.

Die zuvor im Detail erläuterte Plasmadüse wird dazu eingesetzt, den zu desinfizierenden Gegenstand zu überstreichen. Bei dem Gegenstand kann es sich um einen beliebigen Gegenstand handeln, also beispielsweise um ein medizinisches Werkstück, eine Bearbeitungsoberfläche oder einen Lebensmittelbehälter handeln. Während der aus der Plasmadüse austretende Plasmastrahl mit der Oberfläche in Berührung kommt, findet dann die Wechselwirkung des angeregten Plasmagases mit der Oberfläche statt, wodurch die desinfizierende, sterilisierende bzw. entkeimende Wirkung erreicht wird.

## Patentansprüche

1. Verfahren zur Desinfektion von Gegenständen,
- bei dem ein atmosphärischer Plasmastrahl unter Anwendung einer elektrischen Entladung in einem von einem Arbeitgas durchströmten Volumen erzeugt wird und
- bei dem die Oberfläche des Gegenstandes mit dem Plasmastrahl zumindest teilweise beaufschlagt wird,
- wobei die desinfizierende Wirkung durch den Energieübertrag vom Plasmastrahl auf die Oberfläche erfolgt,
**dadurch gekennzeichnet,**
**dass** die elektrische Entladung mittels Anlegen einer hochfrequenten Hochspannung in Form eines Lichtbogens erzeugt wird.

2. Verfahren nach Anspruch 1,
bei dem dem vom Arbeitsgas durchströmten Volumen ein desinfizierender Stoff zugemischt wird.

3. Verfahren nach Anspruch 2,
bei dem als desinfizierender Stoff ein Gas zugemischt wird.

4. Verfahren nach Anspruch 2,
bei dem als desinfizierender Stoff eine Flüssigkeit zugemischt wird.

5. Verfahren nach Anspruch 4,
bei dem der flüssige desinfizierende Stoff in das vom Arbeitsgas durchströmte Volumen eingedüst, versprüht oder vernebelt wird.

6. Verfahren nach einem der Ansprüche 2 bis 5,
bei dem der desinfizierende Stoff strömungsaufwärts der Entladungszone dem Arbeitsgas zugemischt wird.

7. Verfahren nach einem der Ansprüche 2 bis 5,
bei dem der desinfizierende Stoff im Bereich der Entladungszone dem Arbeitsgas zugemischt wird.

8. Verfahren nach einem der Ansprüche 2 bis 5,
bei dem der desinfizierende Stoff strömungsabwärts der Entladungszone dem Arbeitsgas zugemischt wird.

9. Verfahren nach Anspruch 4,
bei dem als flüssiger desinfizierender Stoff Wasser zugemischt wird.

10. Verfahren nach einem der Ansprüche 1 bis 9,
- bei dem zur Erzeugung des Plasmastrahls eine Plasmadüse mit einem Düsenrohr eingesetzt wird, wobei sich das Düsenrohr konisch zu einer Auslassöffnung verjüngt und das Düsenrohr an dem der Auslassöffnung entgegengesetzten Ende eine Dralleinrichtung mit einem Einlass für ein Arbeitsgas aufweist und
- bei dem eine Zwischenwand der Dralleinrichtung einen Kranz von schräg in Umfangsrichtung angestellten Bohrungen aufweist, durch die das Arbeitsgas so verdrallt wird, dass der Lichtbogen im Wirbelkern auf der Achse des Düsenrohrs kanalisiert wird und sich erst im Bereich der Auslassöffnung zur Wand des Düsenrohres verzweigt.

## Claims

1. Method for the disinfection of objects,
- in which an atmospheric plasma jet is produced by utilisation of an electric discharge in a volume through which a process gas flows and
- in which the surface of the object is exposed at least partially to the plasma jet,
- wherein the disinfecting effect occurs due to the energy transfer from the plasma jet to the surface,
**characterized in that**
the electric discharge is produced in form of an arc by application of a high-frequency high voltage.

2. Method according to claim 1,
wherein a disinfecting agent is added into the volume through which the process gas flows.

3. Method according to claim 2,
wherein a gas is added as disinfecting agent.

4. Method according to claim 2,
wherein a liquid is added as disinfecting agent.

5. Method according to claim 4,
wherein the liquid disinfecting agent is injected, sprayed or misted into the volume through which the process gas flows.

6. Method according to one of claims 2 to 5,
wherein the disinfecting agent is mixed into the process gas upstream of the discharge zone.

7. Method according to one of claims 2 to 5,
wherein the disinfecting agent is mixed into the process gas in the area of the discharge zone.

8. Method according to one of claims 2 to 5,
wherein the disinfecting agent is mixed into the process gas downstream of the discharge zone.

9. Method according to claim 4,
wherein water is added as liquid disinfecting agent.

10. Method according to one of claims 1 to 9,
- wherein a plasma nozzle with a nozzle tube is used for producing the plasma jet, wherein the nozzle tube conically tapers towards a nozzle tube outlet and wherein at the end opposite to the nozzle tube outlet the nozzle tube comprises a a twist device having an inlet for a working gas, and
- wherein an intermediate wall of the twist device includes a ring of bores arranged at an angle in the circumferential direction, by which bores the working gas is twisted such, that the arc is channelled in the vortex core along the axis of the nozzle tube and only branches out to the wall of the nozzle tube in the area of the nozzle tube outlet.

## Revendications

1. Procédé pour désinfecter des objets,
- dans lequel un jet de plasma sous pression atmosphérique est généré en utilisant une décharge électrique, dans un volume, qui est traversé par un gaz de travail, et
- dans lequel la surface de l'objet est soumise, tout au moins partiellement, au jet de plasma,
- sachant que l'effet désinfectant est obtenu par la transmission d'énergie du jet de plasma sur la surface, **caractérisé en ce que**
la décharge électrique est générée par application d'une haute tension à haute fréquence, sous la forme d'un arc électrique.

2. Procédé selon la revendication 1, dans lequel une substance désinfectante est ajoutée au volume traversé par le gaz de travail,

3. Procédé selon la revendication 2, dans lequel on utilise un gaz en tant que substance désinfectante.

4. Procédé selon la revendication 2, dans lequel on utilise un liquide en tant que substance désinfectante.

5. Procédé selon la revendication 4, dans lequel la substance désinfectante, liquide est injectée, pulvérisée ou nébulisée dans le volume traversé par le gaz de travail.

6. Procédé selon l'une des revendications 2 à 5, dans lequel la substance désinfectante est ajoutée au gaz de travail en amont de la zone de décharge.

7. Procédé selon l'une des revendications 2 à 5, dans lequel la substance désinfectante est ajoutée au gaz de travail dans la région de la zone de décharge.

8. Procédé selon l'une des revendications 2 à 5, dans lequel la substance désinfectante est ajoutée au gaz de travail en aval de la zone de décharge.

9. Procédé selon la revendication 4, dans lequel de l'eau est ajoutée en tant que substance désinfectante, liquide.

10. Procédé selon l'une des revendications 1 à 9,
- dans lequel, pour générer le jet de plasma, on utilise une buse à plasma avec une lance, sachant que la lance s'amincit, en forme de cône, vers une ouverture de sortie et que ladite lance est dotée, à l'extrémité opposée à l'ouverture de sorte, d'un dispositif de tourbillonnement avec une ouverture d'entrée de gaz de travail, et
- dans lequel, une paroi intermédiaire du dispositif de tourbillonnement est dotée d'une couronne d'alésages, en biais dans la direction périphérique, par lesquels le gaz de travail est amené à tourbillonner de sorte que l'arc électrique soit canalisé, au centre du tourbillon, sur l'axe de la lance et ne se disperse que dans la région de l'ouverture de sortie de la lance.
